# EUROPEAN PATENT APPLICATION

(11) **EP 1 099 447 A2**
(43) Date of publication of application: **16.05.2001**
(21) Application number: 00309939.7
(22) Date of filing: 09.11.2000
(51) Int. Cl.: A61L 15/60

(54) **Hydrogel sheets**

(30) Priority: 11.11.1999 IL 13288099
(71) Applicant: HANITA LENSES, Kibbutz Hanita 22885 (IL)
(72) Inventor: Porat, Menachem, Kibbutz Hanita 22885 (IL)
(74) Representative: Ablewhite, Alan James

(57) **Abstract**

The invention provides a polymeric hydrogel in sheet form comprising a hydrophilic polymer cast within and at least partially surrounding, the mesh of a stretchable material in net form, the material imparting stretchability to the polymeric hydrogel sheet.

## Description

The present invention relates to polymeric hydrogels. More particularly, the present invention relates to polymeric hydrogels in sheet form for use in the medical field and especially for wound dressings and ultrasound and/or electronic monitoring and diagnostics.

As is known and as described, e.g., in U.S. Patent 5,674,346, the relevant teachings of which are incorporated herein by reference, hydrogels are three-dimensional networks of hydrophilic polymers, generally covalently or ionically cross-linked, which interact with aqueous solutions by swelling to some equilibrium value. These cross-linked gels are generally formed from synthetic polymers (such as polyvinylpyrrolidone, polyethyleneoxide, acrylate and methacrylate polymers and copolymers), multivalent alcohols (such as polyvinylalcohol), biopolymers (such as gelatin, agar), or combinations thereof.

Said patent describes the use of hydrogels in the treatment and the management of burns and wounds, in the form of hydrogel dressings and describes the teachings of over 11 patents relating to wound dressing materials incorporating a polymeric hydrogel.

Said patent teaches the preparation of hydrogel laminates, bandages and composits, wherein a hydrogel laminate is formed by providing a substrate coated on at least one surface with at least one adhesive polymer, forming a composite by casting an aqueous solution of at least one hydrophilic polymer onto said coated surface of said substrate, wherein said at least one hydrophilic polymer is copolymerizable with said at least one adhesive polymer and exposing said composite to electron beam irradiation suitable to cross-link said at least one hydrophilic polymer to form said hydrogel and to copolymerize said polymer adhesive and said at least one hydrophilic polymer.

In U.S. Patent 5,817,016, the relevant teachings of which are incorporated herein by reference, there is described and claimed the use of a hydrogel as a coating for a conductor in biomedical electrodes and sensors.

In U.S. Patent 5,522,878, the relevant teachings of which are incorporated herein by reference, there is described and claimed a solid ultrasonic biomedical couplant in sheet form, comprising a hydrogel couplant sheet.

As described in the specification of said latter patent, said sheet includes a solid phase comprising a natural or synthetic hydrophilic polymer which is dispersed in a liquid phase to provide the flexible, solid hydrogel matrix. The liquid phase of the matrix preferably includes water together with a hydrophilic humectant such as a polyhydric alcohol, i.e., one having two or more hydroxyl groups, and as further described therein, in a preferred forming process, the hydrogel is made by coating it while still formable onto a flexible backing sheet, i.e., a liner sheet, usually paper or plastic film.

In the detailed description of the invention found in said patent, it is stated that a solid but flexible gel sheet is provided for trahsferring (coupling) ultrasonic energy to the body. The sheet is composed of a solid phase comprising a synthetic or natural hydrocolloid, such as polyacrylamide or polysaccharide, e.g., a polysaccharide gum or a mixture thereof together with a liquid phase comprising a hydrating agent, e.g., a polyhydric alcohol and water.

The hydrogel sheet of said patent is described as having an exposed skin-contacting surface which is preferably sufficiently tacky to bond to the skin for enhancing the transmission of the ultrasonic signal to the body.

It has now been found that polymeric hydrogel sheets prepared according to said patents, have a major disadvantage in that they are extremely fragile and readily rip and tear. Thus it was found that mere flexibility was not sufficient to provide stability and this is possibly the reason why in U.S. Patent 5,522,878, there is suggested a preferred embodiment, wherein said hydrogel sheet is surrounded by a flexible border and said flexible border comprises a sheet of plastic or rubber.

With this state of the art in mind, there is now provided according to the present invention, a polymeric hydrogel in sheet form comprising a hydrophilic polymer cast within and at least partially surrounding, the mesh of a stretchable material in net form, said material imparting stretchability to said polymeric hydrogel sheet.

In preferred embodiments of the present invention, said material is a stretch dry material such as a mesh of gum or stretch nylon and in especially preferred embodiments, said material is a stretch nylon net such as nylon 6.

In especially preferred embodiments of the present invention, there is provided a polymeric hydrogel in sheet form comprising a hydrophilic polymer polymerized about the mesh of a stretchable material in net form.

In U.S. Patent 4,438,258 there are described hydrogels which may be used as supported or unsupported films and in the description in said patent in column 10, lines 45-48, it is stated that the support may be a contiguous, preferably transparent, backing member or a mesh, for example of nylon, about which the hydrogel has been polymerised.

While said patent which was published in 1984 recites the possible use of a nylon mesh, said patent is silent with regard to the nature of said mesh and from later patents which all use non-stretchable supports for hydrogels, it will be realized that this patent also did not contemplate and did not teach or suggest the use of a stretchable mesh such as taught for the first time in the present invention.

Furthermore, as shown in Comparative Example 3 hereinafter, the use of a nylon mesh which is non-stretchable results during hydration of the hydrogel in the formation of irregularities along the surface of the sheet which prevent the use of such a sheet as an ultrasonic biomedical couplant and, in fact, to the best of Applicant's knowledge, despite the theoretical teaching of U.S. Patent 5,522,878, as of to date there has not yet been provided a commercial hydrogel polymeric sheet which is attachable and utilizable with an ultrasound generator.

Similarly, in US Patent 4,452,892 there is disclosed the immobilization of biologically active material in a hydrogel and on a support whereby some of the hydrogl is enmeshed with the support and the support may be a nylon mesh and in US Patent 5, 648,252 and 5,858,392, there are described supported polyionic hydrogels, however none of said patents teach or suggest the utilization of a stretchable material in net form wherein said stretchable material is also stretchable at its end state and thereby imparts stretchability to the polymeric hydrogel sheet.

The invention will now be described in connection with certain preferred embodiments with reference to the following illustrative figures and examples so that it may be more fully understood.

With specific reference now to the following examples and the accompanying figures, it is stressed that the particulars described and shown are by way of example and for purposes of illustrative discussion of the preferred embodiments of the present invention only and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:
Figure 1 is a cross-sectional view illustrating the preparation of a polymeric hydrogel, according to the present invention;
Figure 2 is an enlarged view of a sheet of stretch material in net form having a polymeric hydrogel incorporated therein;
Figure 3 is a schematic cross-sectional view of the use of a polymeric hydrogel according to the present invention as an ultrasonic or electronic transmission medium;
Figure 4 is a cross-sectional view of an arm of a patient having a polymeric hydrogel wound dressing wound therearound;
Figure 5 is a cross-sectional view of a sheet according to the present invention after polymerization and before final hydration;
Figure 6 is a cross-sectional view of the sheet of Figure 5 according to the present invention after hydration; and
Figure 7 is a cross-sectional view of a comparative sheet not according to the present invention and using a flexible non-stretchable nylon net.

### Example

A stretch pantyhose nylon was wrapped on a plastic block made of hard plastic (Perspex®). The dimension of the plastic and the pantyhose causes enough stretching to prevent any folding of the net nylon on the lower side of the block. The net nylon has a thickness of 0.15 mm.

Solutions for forming a polyacrylamide get were prepared in the same manner as described in example 1 of British patent 2114578, the teachings of which are incorporated herein by reference, i.e., a polyacrylamide gel containing (% by mass) 11 % of polyacrylamide and 89% aqueous solution of sodium chloride was prepared as follows. Three solutions (A, B, C) were previously prepared using the following procedure (amounts of starting components are given for 1000 ml of the basic solution): the solution A was prepared by dissolving 5 ml of tetramethyl ethylenediamine in 995 ml of a 0.5 aqueous solution of sodium chloride and was stored in dark glassware at a temperature of 4°C till the moment of utilization (conventional storage time is 6 to 8 months); the solution B was prepared by dissolving 7.35 g of methylene-bis-acrylamide in 350 ml of a 0.5% aqueous solution of sodium chloride heated up to a temperature of 60°C, following which 280 g of acrylamide was added and mixed until completely dissolved. The solution thus obtained was filtered through a cotton-gauze filter following which a 0.5% aqueous sodium chloride solution was added up to an amount of 1000 ml, and the resulting solution was stored in dark glassware within a refrigerator at a temperature of 4°C till the moment of utilization (conventional storage time is 6 to 8 months); the solution C was prepared by dissolving 1.4 g of ammonium persulfate in 1000 ml of a 0.5% aqueous solution of sodium chloride and was stored in dark glassware till the moment of utilization (conventional storage time is 4 to 6 weeks).

A reaction mixture was prepared from the solutions A, B, C. For this purpose 2 volumetric parts of the solution B and 4 volumetric parts of the solution C were added to 1 volumetric part of the solution A.

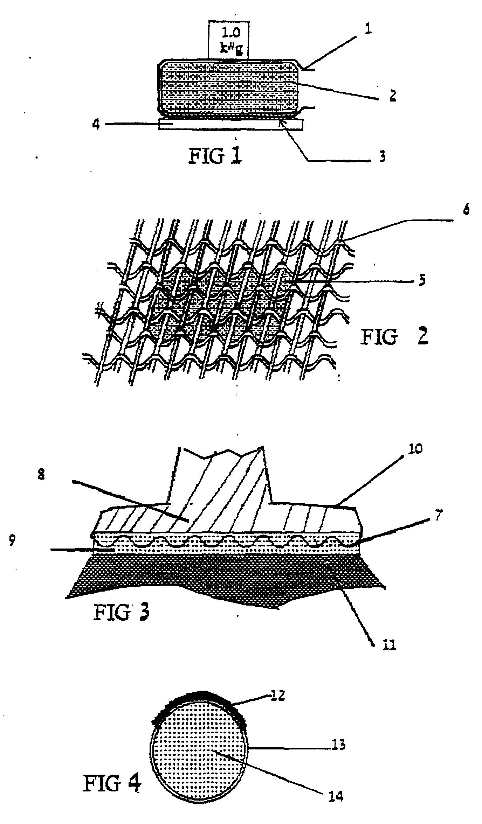

Referring now to figure 1, to better illustrate the further steps of the process utilized for producing the polymeric hydrogels of the present invention, there is seen a sheet of stretchable pantyhose nylon 1, which is used to cover a block of pastic 2, and then placed on the solution 3 prepared above, which solution has been poured onto flat glass 4. The amount of the solution and the gap between the plastic and glass is determined by the desired thickness of the final product. A weight of 1 kg is placed above the nylon-covered plastic block. A thickness of 0.25 mm is achieved without any gap being left between the bottom of the nylon sheet 1 and the glass plate 4.

Polymerization time is approximately 20 minutes at room temperature whereafter the hydrogel 5 which has formed within and at least partially surrounding, the mesh of the net nylon 6, as seen in figure 2, is immersed in distilled water.

### Example 2

Referring to figure 3, there is seen a device 8 used to deliver and/or collect energy to or from the body. The energy can be ultrasound or electricity current. Stretch nylon 7 is seen to be surrounded by the polymeric hydrogel 9, which is strengthened thereby, and which can undergo stretching without being torn.

The hydrogel sheet can have water or saline added thereto, resulting in a smooth interface material without the use of liquid gel, which liquid gel is considered to be very uncomfortable by patients.

Referring to figure 4, there is seen a schematic cross-section of an arm 14 of a patient surrounded by stretch nylon 13, wrapped there around, a portion of which nylon, is provided with a polymeric hydrogel 12 which can be thin or thick according to the patient needs, and which preferably has a thickness, for this use, of about between 6 and 10 mm.

In order to improve the ability for drug delivery, it is possible to provide apertures or tunnels in the hydrogel which can be filled with drug for delivery by methods known per se.

In order to prevent too much evaporation, it is possible to cover the polymer where it is not in contact with the body, with any strip of thin plastic.

### Comparative Example 3

Two polymeric hydrogels in sheet form were prepared according to the procedure of Example 1, however the first was prepared using a stretchable nylon net and the second was prepared using a flexible non-stretchable nylon net such as used in window screens.

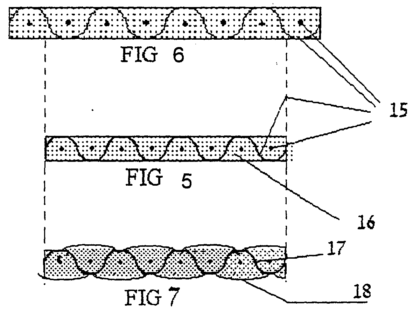

Referring now to Figures 5 and 6, there is seen the net nylon 15 surrounded by polymer 16 after polymerization and before final hydration in Figure 5 and after hydration in Figure 6. As can be seen in Figure 6 the polymer after hydration and water absorption swelled about 130% in three dimensions and the nylon stretched together therewith whereby the surfaces of the sheet remained smooth.

In contradistinction, referring to Figure 7 in which a net 17 was used which was made of tough nylon which could not stretch or swell, the dimension of the whole sheet could not elongate in three dimensions and therefore swelling occurred in the direction of the surfaces, resulting in irregularities 18 which prevent the use of such a sheet as an ultrasonic biomedical couplant.

It will be evident to those skilled in the art that the invention is not limited to the details of the foregoing illustrative embodiments and that the present invention may be embodied in other specific forms without departing from the spirit or essential attributes thereof. The present embodiments are therefore to be considered in all respects as illustrative and not restrictive, the scope of the invention being indicated by the appended claims rather than by the foregoing description, and all changes which come within the meaning and range of equivalency of the claims are therefore intended to be embraced therein.

## Claims

1. A polymeric hydrogel in sheet form comprising a hydrophilic polymer cast within and at least partially surrounding, the mesh of a stretchable material in net form, said material imparting stretchability to said polymeric hydrogel sheet.

2. A polymeric hydrogel according to claim 1, wherein said material is a stretch nylon net.

3. A polymeric hydrogel according to claim 1 comprising a hydrophilic polymer polymerized about the mesh of a stretchable material in net form.
